# EUROPEAN PATENT SPECIFICATION

(11) **EP 0 622 347 B1**
(45) Date of publication and mention of the grant of the patent: **10.06.1998**
(21) Application number: 94103130.4
(22) Date of filing: 02.03.1994
(51) Int. Cl.: C07C 2/36, B01J 31/18, C07C 11/107

(54) **Ethylene trimerisation and catalysts therefor**
Trimerisierung von Ethylen und Katalysatoren dafür
Trimérisation de l'éthylène et catalyseurs pour ce faire

(30) Priority: 03.03.1993 US 25524
(43) Date of publication of application: 02.11.1994
(73) Proprietor: AMOCO CORPORATION, Chicago, IL 60601 (US)
(72) Inventor: Wu, Feng-jung, Baton Rouge, LA 70810 (US)
(74) Representative: Sandmair, Kurt, Dr. Dr.

(56) References cited:
- EP-A- 0 237 079
- EP-A- 0 254 277
- EP-A- 0 531 174
- EP-A- 0 537 609
- US-A- 3 726 939
- US-A- 3 800 000
- US-A- 4 777 315
- JOURNAL OF THE CHEMICAL SOCIETY, CHEMICAL COMMUNICATIONS vol. 1989, no. 11 , LONDON pages 674 - 675 JOHN R. BRIGGS 'The Selective Trimerization of Ethylene to Hex-1-ene'
- JOURNAL OF CATALYSIS vol. 47, no. 2 , 1977 , NEW YORK pages 197 - 209 R.M. MANYIK ET AL. 'A Soluble Chromium-Based Catalyst for Ethylene Trimerization and Polymerization'

## Description

This invention relates generally to the oligomerization of ethylene and more specifically to the preparation of 1-hexene by the trimerization of ethylene using a catalyst which includes an aluminoxane and a chromium complex containing a coordinating polydentate phosphine, stibine or arsine ligand, such as a tridentate phosphine complex of a chromium salt.

Published European patent application, publication number 0,537,609, discloses an ethylene oligomerization/ trimerization process which uses a catalyst comprising a chromium complex which contains a coordinating polydentate ligand and an aluminoxane to produce high quality α-olefins which are enriched in 1-hexene.

Suitable ligands include cyclic polyamines, and polypyrazolyl borates.

US-P-4,777,315 describes a process for the trimerization of an olefin selected from the group consisting of ethylene, propylene, 1-butene and mixtures thereof, comprising passing the olefin and carbon dioxide in contact with a catalyst comprising the reaction product of (i) a chromium compound and (ii) a hydrocarbyl aluminium hydrolyzed with about 0.7 to about 1.1 moles of water per mole of aluminium compound.

EP-A-0 237 079 relates to a process for trimerization of an olefin comprising passing the olefin in contact with a catalyst comprising the reaction product of (i) a chromium compound, (ii) a hydrocarbyl aluminium hydrolyzed with 0.8 to 1.1 moles of water per mole of aluminium compound and (iii) a donor ligand selected from hydrocarbyl isonitriles, amines and ethers.

Briggs et al. describe in J. Chem. Soc. Chem. Comm. **11** (1989), 674-675 a homogeneous three component catalyst of chromium, hydrolysed alkylaluminium and dimethoxyethane for use in the trimerization of ethylene to hexene-1 in 74 % selectivity at a rate of 1.2 mol (mol Cr)⁻¹s⁻¹.

Manyik et al. illustrate in J. Catal. **47(2)** (1977), 197-209 a soluble chromium based catalyst for ethylene trimerization and polymerization, which catalyst is formed by the interaction of chromium (III) 2-ethylhexanoate with partially hydrolyzed triisobutylaluminium.

I have now found that certain polydentate ligand complexes of chromium salts in combination with aluminoxanes can catalyze ethylene oligomerization, and especially ethylene trimerization to form 1-hexene, with a very high degree of selectivity, e.g. about 95%.

In accordance with this invention there is provided a process for the trimerization of ethylene which process comprises reacting ethylene using a catalyst which is as defined in claim 1.

Also provided is an ethylene trimerization catalyst composition comprising an (a) an aluminoxane, and (b) a polydentate phosphine, arsine and/or stibine coordination complex of a chromium salt, wherein said coordination complex has the formula LCrXₙ, wherein L is a coordinating polydentate phosphine, arsine and/or stibine ligand, X represents anions which can be the same or different and n is an integer from 2 to 4.

Aluminoxanes for use in the process of the invention can be prepared as known in the art by reacting water or water containing materials with trialkylaluminum compounds in proportions of from 0.5 to 1.2 equivalents of water and, preferably, 0.8 to 1.0 equivalents of water per equivalent of trialkylaluminum. For example, Manyik et al U.S. 3,300,458 prepare alkylaluminoxane by passing a hydrocarbon solvent through water to form a wet hydrocarbon solvent and mixing the wet hydrocarbon solvent with an alkyl aluminum/ hydrocarbon solvent mixture in a conduit.

Schoenthal et al. U.S. 4,730,071 show the preparation of methylaluminoxane by dispersing water in toluene using an ultrasonic bath and then adding a toluene solution of trimethyl aluminum to the dispersion. Schoenthal et al U.S. 4,730,072 is similar except it uses a high speed, high shear-inducing impeller to form the water dispersion.

Edward et al. U.S. 4,772,736 describe an aluminoxane process in which water is introduced below the surface of a solution of hydrocarbyl aluminum adjacent to a stirrer which serves to immediately disperse the water in the hydrocarbon solution.

The preparation of alkyl aluminoxanes from R₂AlOLi formed by reacting AlR₃ and anhydrous lithium hydroxide, and R₂AlCl has been reported in the literature, for example, Ueyama et al., Inorganic Chemistry, 12, No. 10, 2218 (1973) and Aoyazi et al., Inorganic chemistry, 12, No. 11, 2702 (1973).

Sinn et al. U.S. 4,404,344 prepare methylaluminoxane by adding trimethyl aluminum to a slurry of CuSO₄•5H₂O in toluene. Introducing water as a metal hydrate controls its reactivity with the trimethyl aluminum. Kaminsky et al. U.S. 4,544,762 is similar except it uses an aluminum sulfate salt hydrate to supply the water. Likewise, Welborn et al. U.S. 4,665,208 describe the use of other metal salt hydrates such as FeSO₄•7H₂O as a water source in preparing aluminoxane.

Hydrocarbylaluminoxanes may exist in the form of linear or cyclic polymers with the simplest compounds being a tetraalkylaluminoxane such as tetraethylaluminoxane, (C₂H₅)₂AlOAl(C₂H₅)₂. Preferred aluminoxanes are prepared from trialkyl aluminum compounds such as triethyl aluminum, tri-n-butyl aluminum, triisobutyl aluminum, tri-n-hexyl aluminum, and tri-octyl aluminum. Of these, the more preferred are the compounds having C₆ or higher alkyl groups which have better solubility in the hydrocarbon solvent reaction medium. The aluminoxanes used to form the catalyst are preferably contained in organic solvents in concentrations of from 0.3 to 30 weight percent of total solvent plus aluminoxane.

A trialkylaluminum compound can also be included in the catalyst (0.1 to 1.0 mole per mole of aluminoxane).

The chromium complexes which, upon mixing with an aluminoxane, catalyze ethylene oligomerization and especially trimerization in accordance with the process of the invention are represented by the formula: LCrXₙ, wherein L is a coordinating polydentate phosphine, arsine and/or stibine ligand and X represents anions which can be the same or different and n is an integer of 2 to 4. Such complexes can be in the form of oligomers, i.e. (LCrXₙ)_{y} where y is 2 to 8. By "polydentate" is meant that the ligand contains multiple donor atoms for coordination with chromium.

Preferred polydentate ligands include the following types:
(a) RY(R'ZR₂")₂
   wherein R and R" are hydrogen or C₁ to C₂₀ hydrocarbyl; R' is C₁ to C₁₀ hydrocarbyl; and Y and Z are individually phosphorus, arsenic or antimony;
(b) CH₃E(R'ZR₂")₃
   wherein E is C,Si, Ge or Sn and R', R" and Z are as defined in (a) above;
(c) E'(R'ZR₂'')₃
   wherein E' is nitrogen, phosphorus, arsenic or antimony and R', R'' and Z are as defined in (a) above; and
(d) A-ZR-B
   wherein A is an integer of 9 to 18, B is an integer of 3 to 6, R is a C₁ to C₁₀ alkyl group such as a methyl, ethyl, propyl, butyl, pentyl, hexyl or higher alkyl group or a C₆ to C₂₀ aromatic group such as benzyl and Z is phosphorous, arsenic or antimony. The abbreviations, such as 9-PR-3, 10-PR-3, 12-PR-4 and the like, used for the phosphine ligands correspond to those used for crown ethers because they are their phosphorus analogues. For example, 9-PR-3 denotes a nine membered ring with 3 phosphorus atoms. The most preferred coordinating polydentate ligands of this type are facially coordinating tridentate ligands such as 9-PMe-3.

In the ligands of types (a), (b) and (c) each (R'ZR") moiety can be different so as to provide a mixture of donors in the same complex. The ligands of types (a), (b), (c), and (d) can be modified to attach to a polyethylene chain (m wt. = 1000 or higher) so that the resulting catalyst is homogeneous at elevated temperature but becomes heterogeneous at 25°C. This technique facilitates the recovery of the catalyst from the reaction products for reuse and has been used with other catalysts as described, for example, by D. E. Bergbreiter et al., J. Chem. Soc., Chem. Commun., 337-338 (1985); J. Org. Chem. (1986) 51, 4752-4760; and J.A.C.S. (1987), 109, 177-179.

Non-limiting examples of specific tridentate phosphine ligands include:
for type (a), EtP(C₂H₄PEt₂)₂, whose chemical name is bis-(2-diethylphosphinoethyl)ethylphosphine;
for type (b), CH₃C(CH₂PEt₂)₂, whose chemical name is 1,1,1-tris(diethylphosphinomethyl)ethane;
for type (c), P(C₂H₄PEt₂)₃, whose chemical name is tris(2-diethylphosphinoethyl)phosphine; and
for type (d), 9-PMe-3, whose chemical name is 1,4,7-trimethyl-1,4,7-triphosphino-cyclononane.

Other specific examples are:
CH₃C(CH₂PPh₂)₃
PhP(CH₂CH₂PPh₂)₂
CyP(CH₂CH₂PCy₂)₂
CyP(CH₂CH₂PEt₂)₂
n-PrP(CH₂CH₂PEt₂)₂
EtP(C₃H₆PEt₂)₂
N(C₂H₄PEt)₃
PhP(o-C₆H₄PEt₂)₂

wherein Ph = phenyl, Cy = cyclohexyl, Me = methyl, Et = ethyl and Pr = propyl. The arsine and stibine analogues of these ligands could also be prepared, for example:
PhAs(o-C₆H₄AsPh₂)₂
MeAs(o-C₆H₄AsMe₂)₂
MeSb(C₂H₄SbMe₂)₂
MeAs(C₃H₆AsMe₂)₂

By a coordinating polydentate ligand is meant a ligand that sterically encumbers the chromium atom in such a way that the rate of chain propagation is decreased so that oligomeriza-tion, especially trimerization, rather than polymerization occurs. For example, ligands which occupy three adjacent coordination sites about an octahedral chromium atom.

Examples of suitable anions, X, include, but are not limited to, halides (Cl⁻, Br⁻, I⁻, F⁻), alkoxides (OR⁻), carboxylates (O₂CR⁻), and Oxo(O⁻²). These anions are initially the anion portion of the chromium compounds used to make the complex. The chromium in the compounds is initially in the oxidation state of II to VI and is preferably in the oxidation state of II, III or IV.

The chromium complexes can be prepared according to procedures set forth in the literature. For example L. R. Gray et al., J. Chem. Soc. Dalton. Trans. (1984), 47-53 and A. M. Arif et al. Inorg. Chem., Vol. 25, No. 8, 1986, 1080 - 1084.

The chromium complex and aluminoxane are combined in proportions to provide Al/Cr molar ratios of from 1:1 to 10,000 to 1 and, preferably, from 5:1 to 500 to 1. The amount of catalyst used is selected to provide the desired reaction rates at any particular reaction scale. (The presence of amounts of 0.001 mmole or more and preferably from 0.1 to 10 mmoles of chromium catalyst in a 300 ml reactor are effective to catalyze the reaction.) Catalyst mixing is preferably done at low temperatures of 0 to 35°C. The presence of ethylene during catalyst mixing at these temperatures resulted in no significant difference in catalyst properties when compared with catalysts prepared in the absence of ethylene. Ethylene provided a protective effect at temperatures above 55°C.

The reaction with ethylene is carried out in an inert solvent. Any inert solvent which does not react with aluminoxane can be used. The preferred solvents are aliphatic and aromatic hydrocarbons and halogenated hydrocarbons such as, for example, toluene, xylene, ethylbenzene, cumene, mesitylene, heptane, cyclohexane, methylcyclohexane, 1-hexene, 1-octene, chlorobenzene, and dichlorobenzene. The amount of solvent is not particularly critical and generally ranges from 50 to 99 wt. percent of the initial reaction mixture.

Reaction temperatures and pressures are chosen to optimize reaction rates and selectivity. In general temperatures of from 35 to 200°C are used and preferably 80 to 120°C. Ethylene pressures can range from atmospheric to (3000 psig) 20,786 kPa and preferably from (100 to 1500 psig) 791-10,444 kPa). Temperature and pressure affect reaction rate and purity in the following way: both higher temperature and higher ethylene pressure increase reaction rate; higher ethylene pressures give better purity by forming less internal olefins, whereas higher temperatures increase the formation of internal olefins.

The invention is further illustrated by, but is not intended to be limited to, the following examples.

### Example 1 - Preparation of Triphosphine Chromium Trichloride

### Preparation of n-PrP(CH=CH₂)₂

To a 1.0 M solution of vinylMgBr (70 mmol) in THF at 0°C was added a solution of n-PrPCl₂ (3.75 g, 25.9 mmol) in 35 ml THF over 1 hour. The solution was allowed to warm slowly and stirred overnight. To the resulting suspension was added degassed saturated NH₄Cl solution (50 ml) slowly to kill the unreacted vinylMgBr. The organic phase was separated from the aqueous phase using a cannula. The remaining aqueous phase was washed with two 40-ml portions of Et₂O, which were then combined with the organic phase, dried over sodium carbonate and distilled at ambient pressure under inert atmosphere to give 2.0 g (60% yield) of n-PrP(C₂H₃)₂ (b.p. = 143°C).

### Preparation of n-PrP(C₂H₄PEt₂)₂

A mixture of n-PrP(CH=CH₂)₂ (1.29 g, 10.0 mmol), Et₂PH (2.25 g, 25.0 mmol) and 2,2'-azobis(isobutyronitrile) (AIBN, 30 mg) in a closed flask under inert atmosphere was irradiated by a GE Sunlamp (275 W) one foot away for 24 hours. The resulting colorless liquid was stripped of volatiles under vacuum and vacuum distilled to give 3.1 g (97% yield) of product collected at 132-135°C/0.35 mmHg (46.7 Pa). ³¹P-NMR (toluene): δ - 18.5 (2P); δ - 22.8 (1P).

### Preparation of [n-PrP(C₂H₄PEt₂)₂]CrCl₃

A mixture of n-PrP(C₂H₄PEt₂)₂ (2.30 g, 7.46 mmol) and anhydrous CrCl₃ (0.40 g, 2.50 mmol) in a closed flask under vacuum was heated with stirring at 135 °C for 1 hour. The reaction mixture at this stage contained four compounds: excess ligand (heptane-soluble), purple LCrCl₃ (toluene-soluble), blue LCrCl₃ (CH₂Cl₂-soluble), and unreacted CrCl₃. Separation was achieved by solubility difference. The resulting blue cake was extracted with 20 ml of toluene, filtered, and washed with toluene until colorless. Toluene was removed from the combined purple filtrate, the residue was extracted with heptane, filtered to give a purple solid and unreacted ligand in heptane. The insoluble materials were a mixture of a blue solid and unreacted CrCl₃. Separation was achieved by extraction with CH₂Cl₂. Unreacted CrCl₃ (0.05 g) was recovered. Results: blue solid; 0.65 g, purple solid; 0.35 g. The combined yield was quantitative based on reacted CrCl₃. The blue and purple solids are both active in the ethylene trimerization reaction. Anal. for the blue compound, Calcd: P, 19.91; Cl, 22.79; Cr, 11.14; C, 38.60; H, 7.56. Found P, 19.77; Cl, 23.14; Cr, 11.46; C, 38.20; H 7.65.

The following diagram shows the structure of the product:

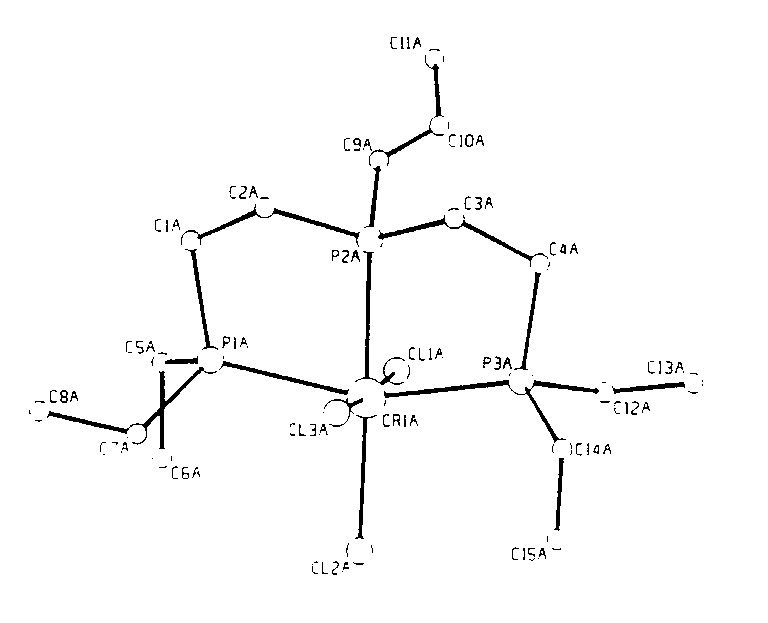

Another ligand-chromium complex, [CyP(C₂H₄PEt₂)₂]CrCl₃, where Cy is cyclohexyl, was prepared analogously.

### Example 2 - Ethylene Trimerization Reaction

The reaction was carried out in a 300 ml Parr stainless-steel reactor to which a liquid addition bomb was connected for the purpose of adding the aluminoxane solution under ethylene pressure. To the reactor containing a solution of [n-PrP(C₂H₄PEt₂)₂]CrCl₃ (45 mg, 0.096 mmol) and pentadecane (0.267 g, as internal reference for gas chromatography) in 90 ml of toluene at 25°C under (250 psig) 1825 kPa of ethylene pressure was added a solution of n-hexylaluminoxane (5.0 mmol) in 10 ml of toluene using ethylene gas which brought the pressure to (300 psig) 2197 kPa . The chain-growth reaction was then carried out with continuous ethylene feed at 95°C 4302 kPa (610 psig) for one hour (stirring rate: 800 RPM), during which time 22 g of ethylene was consumed. The reaction was terminated by pressing methanol into the reactor to deactivate the catalyst. The reactor was cooled to 10°C, vented, and a sample was withdrawn for GC analysis which showed the following results: C₄: 4.3%, C₆: 94.3%, C₈: 0.2%, C₁₀: 0.9%. The purity of 1-hexene was 92.6% with major impurities being internal hexenes. The weights of the carbon fractions were calculated using measured response factors and mimic experiments to simulate the operational loss of light olefins.

Results of this and other Examples 3-8 with varied reaction conditions are summarized in Table I.

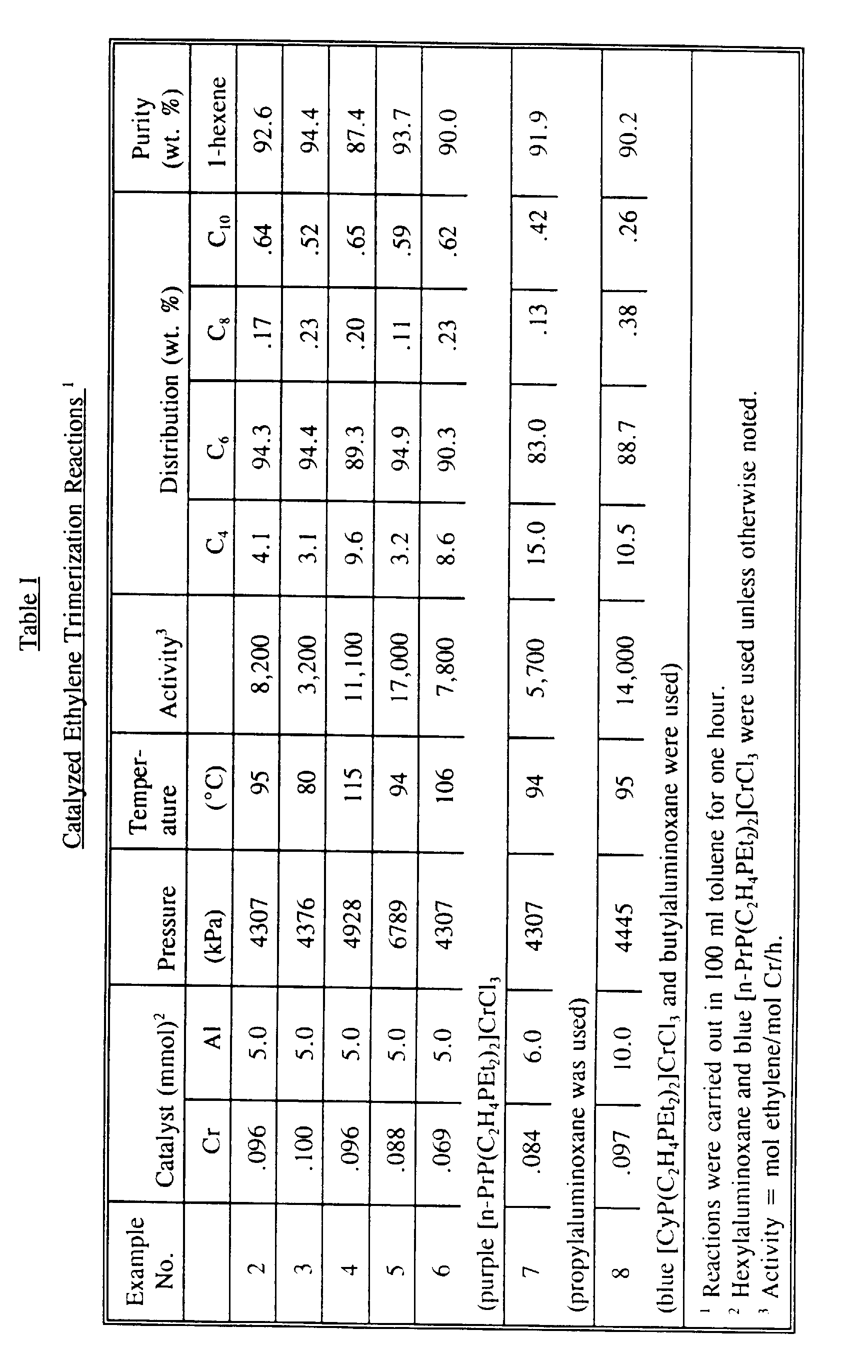

## Claims

1. A process for the trimerization of ethylene, comprising oligomerizing ethylene in the presence of an ethylene trimerization catalyst composition comprising (a) an aluminoxane, and (b) a polydentate phosphine, arsine and/or stibine coordination complex of a chromium salt, so as to selectively from 1-hexene without substantial formation of polyethylene, wherein said coordination complex has the formula LCrXₙ, wherein L is a coordinating polydentate phosphine, arsine and/or stibine ligand, X represents anions which can be the same or different and n is an integer from 2 to 4.

2. The process according to Claim 1, wherein the polydentate ligand in the coordination complex has the formula:
RY(R'ZR₂")₂
wherein R' and R" are hydrogen or C₁ to about C₂₀ hydrocarbyl; R' is C₁ to about C₁₀ hydrocarbyl; and Y and Z are individually phosphorus, arsenic or antimony.

3. The process according to Claim 1, wherein the polydentate ligand in the coordination complex has the formula:
CH₃E(R'ZR₂")₃
wherein E is C, Si, Ge or Sn; R¹ is C₁ to about C₂₀ hydrocarbyl; each R" is individually hydrogen or C₁ to about C₁₀ hydrocarbyl, and each Z is individually phosphorus, arsenic or antimony.

4. The process according to Claim 1, wherein the polydentate ligand in the coordination complex has the formula:
E'(R'ZR₂")₃
wherein E' is nitrogen, phosphorus, arsenic or antimony; R' is C₁ to about C₂₀ hydrocarbyl; each R" is individually hydrogen or C₁ to about C₁₀ hydrocarbyl and each Z is individually phosphorus, arsenic or antimony.

5. The process according to Claim 1, wherein the polydentate ligand in the coordination complex has the formula:
A-ZR-B
wherein A is an integer of 9 to 19, B is an integer of 3 to 6, R is a C₁ to C₁₀ alkyl or a C₆ to C₂₀ aryl group, and Z is phosphorus, arsenic or antimony.

6. The ethylene trimerization catalyst composition comprising (a) an aluminoxane, and (b) a polydentate phosphine, arsine and/or stibine coordination complex of a chromium salt, wherein said coordination complex has the formula LCrXₙ, wherein L is a coordinating polydentate phosphine, arsine and/or stibine ligand, X represents anions which can be the same or different and n is an integer from 2 to 4.

7. The ethylene trimerization catalyst composition wherein the polydentate ligand in the coordination complex has the formula:
RY(R'ZR₂")₂
wherein R' and R'' are hydrogen or C₁ to about C₂₀ hydrocarbyl; R' is C₁ to about C₁₀ hydrocarbyl: and Y and Z are individually phosphorus, arsenic or antimony of Claim 6.

8. The ethylene trimerization catalyst composition of Claim 7, wherein the polydentate ligand in the coordination complex has the formula:
CH₃E(R'ZR₂")₃
wherein E is C, Si, Ge or Sn; R¹ is C₁ to about C₂₀ hydrocarbyl; each R" is individually hydrogen or C₁ to about C₁₀ hydrocarbyl, and each Z is individually phosphorus, arsenic or antimony.

9. The ethylene trimerization catalyst composition of Claim 8, wherein the polydentate ligand in the coordination complex has the formula:
E'(R'ZR₂")₃
wherein E' is nitrogen, phosphorus, arsenic or antimony; R' is C₁ to about C₂₀ hydrocarbyl; each R" is individually hydrogen or C₁ to about C₁₀ hydrocarbyl, and each Z is individually phosphorus, arsenic or antimony.

10. The ethylene trimerization catalyst composition of Claim 9, wherein the polydentate ligand in the coordination complex has the formula:
A-ZR-B
wherein A is an integer of 9 to 19, B is an integer of 3 to 6, R is a C₁ to C₁₀ alkyl or a C₆ to C₂₀ aryl group, and Z is phosphorus, arsenic or antimony.

## Patentansprüche

1. Verfahren zur Trimerisierung von Ethylen, bei dem man Ethylen in Gegenwart einer (a) ein Aluminoxan und (b) einen vielzähnigen Phosphin-, Arsin- und/ oder Stibinkoordinationskomplex eines Chromsalzes enthaltenden Ethylentrimerisierungs-Katalysatorzusammensetzung oligomerisiert, um selektiv 1-Hexen ohne wesentlichen Bildung von Polyethylen herzustellen, wobei der Koordinationskomplex die Formel LCrXₙ aufweist, in der L ein koordinierender vielzähniger Phosphin-, Arsin- und/oder Stibinligand ist, X Anionen darstellt, die gleich oder voneinander verschieden sein können, und n eine ganze Zahl von 2 bis 4 ist.

2. Verfahren nach Anspruch 1, bei dem der vielzähnige Ligand im Koordinationskomplex die Formel
RY(R'ZR₂")₂
hat, in der R' und R" Wasserstoff oder C₁- bis etwa C₂₀-Hydrocarbyl bedeuten, R' C₁- bis etwa C₁₀-Hydrocarbyl ist und Y und Z individuell Phosphor, Arsen oder Antimon bedeuten.

3. Verfahren nach Anspruch 1, bei dem der vielzähnige Ligand im Koordinationskomplex die Formel
CH₃E(R'ZR₂")₃
hat, in der E C, Si, Ge oder Sn ist, R' C₁- bis etwa C₂₀-Hydrocarbyl bedeutet, jedes R" individuell Wasserstoff oder C₁- bis etwa C₁₀-Hydrocarbyl ist und jedes Z individuell Phosphor, Arsen oder Antimon bedeutet.

4. Verfahren nach Anspruch 1, bei dem der vielzähnige Ligand im Koordinationskomplex die Formel
E'(R'ZR₂")₃
hat, in der E' Stickstoff, Phosphor, Arsen oder Antimon ist, R' C₁- bis etwa C₂₀-Hydrocarbyl bedeutet, jedes R" individuell Wasserstoff oder C₁-bis etwa C₁₀-Hydrocarbyl ist und jedes Z individuell Phosphor, Arsen oder Antimon bedeutet.

5. Verfahren nach Anspruch 1, bei dem der vielzähnige Ligand im Koordinationskomplex die Formel
A-ZR-B
hat, in der A eine ganze Zahl von 9 bis 19, B eine ganze Zahl von 3 bis 6, R eine C₁-C₁₀-Alkyl- oder eine C₆-C₂₀-Arylgruppe bedeutet und Z Phosphor, Arsen oder Antimon ist.

6. Ethylentrimerisierungs-Katalysatorzusammensetzung, umfassend (a) ein Aluminoxan und (b) einen vielzähnigen Phosphin-, Arsin- und/oder Stibinkoordinationskomplex eines Chromsalzes, wobei der Koordinationskomplex die Formel LCrXₙ aufweist, in der L ein koordinierender vielzähniger Phosphin-, Arsin- und/oder Stibinligand ist, X Anionen darstellt, die gleich oder voneinander verschieden sein können, und n eine ganze Zahl von 2 bis 4 ist.

7. Ethylentrimerisierungs-Katalysatorzusammensetzung, in der der vielzähnige Ligand im Koordinationskomplex die Formel
RY(R'ZR₂")₂
hat, in der R' und R" Wasserstoff oder C₁- bis etwa C₂₀-Hydrocarbyl bedeuten, R' C₁- bis etwa C₁₀-Hydrocarbyl ist und Y und Z individuell Phosphor, Arsen oder Antimon nach Anspruch 6 bedeuten.

8. Ethylentrimerisierungs-Katalysatorzusammensetzung nach Anspruch 7, in der der vielzähnige Ligand im Koordinationskomplex die Formel
CH₃E(R'ZR₂")₃
hat, in der E C, Si, Ge oder Sn ist, R' C₁- bis etwa C₂₀-Hydrocarbyl bedeutet, jedes R" individuell Wasserstoff oder C₁- bis etwa C₁₀-Hydrocarbyl ist und jedes Z individuell Phosphor, Arsen oder Antimon bedeutet.

9. Ethylentrimerisierungs-Katalysatorzusammensetzung nach Anspruch 8, in der der vielzähnige Ligand im Koordinationskomplex die Formel
E'(R'ZR₂")₃
hat, in der E' Stickstoff, Phosphor, Arsen oder Antimon ist, R' C₁- bis etwa C₂₀-Hydrocarbyl bedeutet, jedes R" individuell Wasserstoff oder C₁-bis etwa C₁₀-Hydrocarbyl ist und jedes Z individuell Phosphor, Arsen oder Antimon bedeutet.

10. Ethylentrimerisierungs-Katalysatorzusammensetzung nach Anspruch 9, in der der vielzähnige Ligand im Koordinationskomplex die Formel
A-ZR-B
hat, in der A eine ganze Zahl von 9 bis 19, B eine ganze Zahl von 3 bis 6, R eine C₁-C₁₀-Alkyl- oder eine C₆-C₂₀-Arylgruppe bedeutet und Z Phosphor, Arsen oder Antimon ist.

## Revendications

1. Procédé de trimérisation de l'éthylène, comprenant l'oligomérisation d'éthylène en présence d'une composition de catalyseur de la trimérisation de l'éthylène comprenant (a) un aluminoxane, et (b) un complexe de coordination de phosphine, d'arsine et/ou de stibine polydentées et d'un sel de chrome, de manière à sélectivement former du 1-hexène sans forte formation de polyéthylène, ledit complexe de coordination ayant la formule LCrXₙ, dans laquelle L est un ligand de coordination polydenté de phosphine, d'arsine et/ou de stibine, X représente des anions qui peuvent être identiques ou différents et n est un nombre entier de 2 à 4.

2. Procédé suivant la revendication 1, dans lequel le ligand polydenté du complexe de coordination a la formule :
RY(R'ZR₂")₂
dans laquelle R' et R" sont l'hydrogène ou un hydrocarbyle en C₁ à environ C₂₀; R' est un hydrocarbyle en C₁ à environ C₁₀, et Y et Z sont individuellement un phosphore, un arsenic ou un antimoine.

3. Procédé suivant la revendication 1, dans lequel le ligand polydenté du complexe de coordination a la formule :
CH₃E(R'ZR₂")₃
dans laquelle E est C, Si, Ge ou Sn; R¹ est un hydrocarbyle en C₁ à environ C₂₀; chaque R" est individuellement un hydrogène ou un hydrocarbyle en C₁ à environ C₁₀, et chaque Z est individuellement un phosphore, un arsenic ou un antimoine.

4. Procédé suivant la revendication 1, dans lequel le ligand polydenté du complexe de coordination a la formule :
E'(R'ZR₂")₃
dans laquelle E' est un azote, un phosphore, un arsenic ou un antimoine; R¹ est un hydrocarbyle en C₁ à environ C₂₀; chaque R" est individuellement un hydrogène ou un hydrocarbyle en C₁ à environ C₁₀, et chaque Z est individuellement un phosphore, un arsenic ou un antimoine.

5. Procédé suivant la revendication 1, dans lequel le ligand polydenté du complexe de coordination a la formule :
A-ZR-B
dans laquelle A est un nombre entier de 9 à 19, B est un nombre entier de 3 à 6, R est un alcoyle en C₁₋₁₀ ou un radical aryle en C₆₋₂₀, et Z est un phosphore, un arsenic ou un antimoine.

6. Composition de catalyseur de trimérisation de l'éthylène comprenant (a) un aluminoxane, et (b) un complexe de coordination de phosphine, d'arsine et/ou de stibine polydentée et d'un sel de chrome, ledit complexe de coordination ayant la formule LCrXₙ, dans laquelle L est un ligand de coordination polydenté de phosphine, d'arsine et/ou de stibine, X représente des anions qui peuvent être identiques ou différents et n est un nombre entier de 2 à 4.

7. Composition de catalyseur de trimérisation de l'éthylène dans laquelle le ligand polydenté du complexe de coordination a la formule :
RY(R'ZR₂")₂
dans laquelle R' et R" sont l'hydrogène ou un hydrocarbyle en C₁ à environ C₂₀; R' est un hydrocarbyle en C₁ à environ C₁₀, et Y et Z sont individuellement un phosphore, un arsenic ou un antimoine de la revendication 6.

8. Composition de catalyseur de trimérisation de l'éthylène suivant la revendication 7, dans laquelle le ligand polydenté du complexe de coordination a la formule :
CH₃E(R'ZR₂")₃
dans laquelle E est C, Si, Ge ou Sn; R¹ est un hydrocarbyle en C₁ à environ C₂₀; chaque R" est individuellement un hydrogène ou un hydrocarbyle en C₁ à environ C₁₀, et chaque Z est individuellement un phosphore, un arsenic ou un antimoine.

9. Composition de catalyseur de trimérisation de l'éthylène suivant la revendication 8, dans laquelle le ligand polydenté du complexe de coordination a la formule :
E'(R'ZR₂")₃
dans laquelle E' est un azote, un phosphore, un arsenic ou un antimoine; R¹ est un hydrocarbyle en C₁ à environ C₂₀; chaque R" est individuellement un hydrogène ou un hydrocarbyle en C₁ à environ C₁₀, et chaque Z est individuellement un phosphore, un arsenic ou un antimoine.

10. Composition de catalyseur de trimérisation de l'éthylène suivant la revendication 9, dans laquelle le ligand polydenté du complexe de coordination a la formule :
A-ZR-B
dans laquelle A est un nombre entier de 9 à 19, B est un nombre entier de 3 à 6, R est un alcoyle en C₁₋₁₀ ou un radical aryle en C₆₋₂₀, et Z est un phosphore, un arsenic ou un antimoine.
